(19) Europäisches Patentamt
European Patent Office
Office européen des brevets

(11) Numéro de publication: 0 092 031
B1

(12) **FASCICULE DE BREVET EUROPEEN**

(45) Date de publication du fascicule du brevet: **27.12.84**

(51) Int. Cl.³: **C 07 C 47/225**, C 07 C 45/58, C 11 B 9/00, A 61 K 7/46

(21) Numéro de dépôt: **83101316.4**

(22) Date de dépôt: **11.02.83**

(54) **1(7)-p-Menthène-9-al et son utilisation en tant qu'ingrédient parfumant et aromatisant.**

(30) Priorité: **15.04.82 CH 2280/82**

(43) Date de publication de la demande: **26.10.83 Bulletin 83/43**

(45) Mention de la délivrance du brevet: **27.12.84 Bulletin 84/52**

(84) Etats contractants désignés: **CH DE FR GB LI NL**

(56) Documents cités:
FR - A - 1 294 933
GB - A - 1 009 916
US - A - 2 710 825

JOURNAL OF ORGANIC CHEMISTRY, vol. 42, no. 21, 1977, pages 3360-3364

(73) Titulaire: **FIRMENICH SA, 1, route des Jeunes, CH-1211 Genève 8 (CH)**

(72) Inventeur: **Morris, Anthony Francis, Route de Trélex, CH-1261 Gingins (CH)**
Inventeur: **Delay, François, Dr., 9, rue des Pervenches, CH-1227 Carouge (CH)**
Inventeur: **Gautschi, Fritz, Dr., Chemin de la Faucille, CH-1299 Commugny (CH)**
Inventeur: **Thomas, Alan Francis, Dr., Chemin de Grens, CH-1261 Borex (CH)**
Inventeur: **Giersch, Wolfgang, Klaus, 353, Route de Bernex, CH-1233 Bernex (CH)**
Inventeur: **Boschung, André, Dr., Les Condémines, CH-1181 Gilly (CH)**

(74) Mandataire: **Salvadori, Giuseppe, Dr., c/o Firmenich S.A. Case Postale 239, CH-1211 Genève 8 (CH)**

EP 0 092 031 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a trait au domaine de la parfumerie et des arômes, elle concerne en particulier l'utilisation d'un aldéhyde monoterpénique nouveau, le 1(7)-p-menthène-9-al en tant qu'ingrédient parfumant et aromatisant.

Le 1(7)-p-menthène-9-al est un composé représenté par la formule suivante:

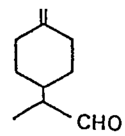
(I)

quoique apparenté à son isomère connu ayant une double liaison endocyclique, le 1-p-menthène-9-al, ledit composé de formule I est une entité chimique nouvelle. Ses propriétés organoleptiques sont fort intéressantes et par conséquent il trouve une utilisation étendue dans des domaines variés de la parfumerie et des arômes.

Le composé I possède en effet une odeur amère de feuille verte et présente un caractère herbal et aromatique. Cette fragrance est tout à la fois fraîche et montante et particulièrement puissante.

Dans le domaine des arômes, le 1(7)-p-menthène-9-al se distingue par son caractère fleuri, herbal et gras qui rappelle, par certains de ses côtés, l'arôme de noix ou le cumin.

Le 1-p-menthène-9-al présente par contre une note olfactive verte et grasse, note qui rappelle, tout au moins en partie, le caractère propre de l'essence de gingergrass. Ce même composé possède par ailleurs une odeur fleurie, herbale, douce et lactonique, voire coumarinée. Au vu de ces caractères, le 1-p-menthène-9-al ne peut valablement constituer un substitut du composé I de l'invention dans les domaines d'utilisation envisagés.

Les proportions dans lesquelles le 1(7)-p-menthène-9-al est utilisé pour produire les effets odorants désirés peuvent varier dans une gamme de valeurs assez étendue. Sa puissance est telle cependant que des concentrations de l'ordre de 0,01 à 0,1-0,5% en poids, par rapport au poids de la composition dans laquelle il est incorporé, peuvent déjà produire un effet marqué.

Ces proportions peuvent être de l'ordre de quelques parties par million lors de l'emploi du composé de l'invention dans le domaine des arômes.

Un dosage moyen de 1 à 10 ppm est suffisant dans la plupart des applications envisagées. De par ses propriétés aromatisantes, le composé de l'invention est particulièrement adapté à l'aromatisation de boissons, d'infusions et du tabac.

Le 1(7)-p-menthène-9-al est préparé suivant l'invention par un procédé qui consiste

a) à traiter le 7,8-dihydroxy-p-menthane avec un agent d'acétylation pour fournir le 7-acétoxy-8-hydroxy-p-menthane;
b) à déshydrater ce dernier afin d'obtenir le 7-acétoxy-8-p-menthène;
c) à époxyder le composé ainsi obtenu pour donner le 7-acétoxy-8,9-époxy-p-menthane, et
d) à soumettre enfin cet époxyde à un traitement thermique pour fournir un pyrolysat constitué pour l'essentiel du 1(7)-p-menthène-9-al désiré.

Les différentes étapes distinctes du procédé décrit font appel à des réactions de type connu. C'est ainsi que l'étape a est effectuée à l'aide d'un agent d'acétylation ordinaire, par exemple l'anhydride acétique en présence de pyridine.

La déshydratation qui suit est conduite à l'aide d'alumine. Selon un mode opératoire préférentiel, on effectue ladite déshydratation en faisant passer une solution de l'hydroxyacétate de départ, dans un solvant organique inerte, à travers une colonne remplie de γ-alumine mélangée à du sable de quartz, colonne préalablement chauffée à 250°C. Le condensat obtenu par refroidissement des vapeurs formées est ensuite époxydé par exemple à l'aide d'un peracide. A cet effet, l'acide peracétique est utilisé de préférence.

La dernière étape du procédé décrit consiste en un traitement thermique de l'époxyacétate obtenu. Bien entendu, cette opération peut être conduite à des températures pouvant varier dans une gamme de valeurs assez large, par exemple comprises entre 400 et 550°C. Comme souvent en pareil cas, aux températures inférieures aux limites indiquées, les temps de réaction sont assez lents, tandis qu'en opérant à des températures supérieures à celles indiquées ci-dessus, il devient difficile de contrôler la formation de produits secondaires non désirés. Le pyrolysat obtenu en refroidissant les vapeurs formées est constitué éminemment par le 1(7)-p-menthène-9-al désiré accompagné par les deux composés de formules

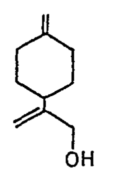
et
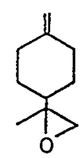

Ces deux derniers composés, par ailleurs, ne semblent pas exercer une influence négative sur l'odeur ou le goût propre de l'aldéhyde en question. Par conséquent, la purification du mélange directement issu du procédé décrit n'est pas indispensable. Cependant, au cas où une séparation de l'aldéhyde pur serait tout de même souhaitée, l'on pourra effectuer celle-ci à l'aide d'un simple fractionnement.

Le procédé de l'invention est illustré par le schéma réactionnel suivant:

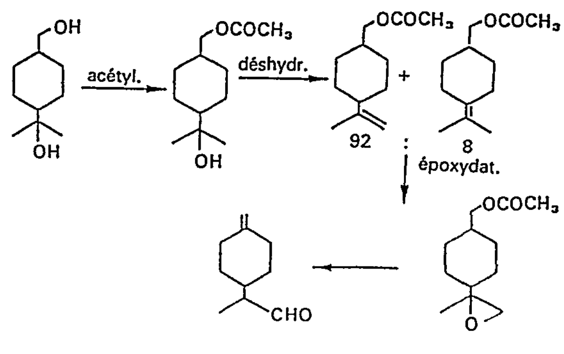

Le 7,8-dihydroxy-p-menthane, utilisé comme produit de départ dans le procédé de l'invention, peut être préparé à partir de β-pinène suivant un procédé qui peut être illustré ainsi:

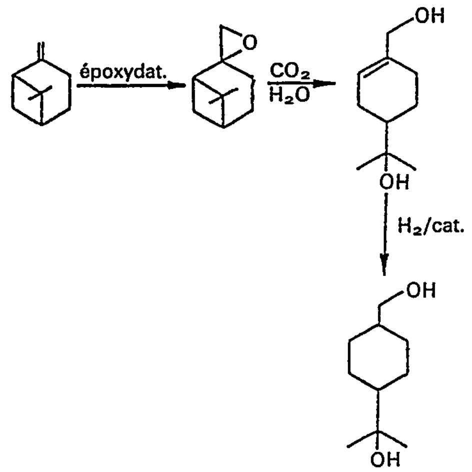


L'invention est illustrée de manière plus détaillée par les exemples suivants, dans lesquels les températures sont indiquées en degrés centigrades et les abréviations ont le sens usuel dans l'art.

*Exemple 1:*

*Préparation de 1(7)-p-menthène-9-al*

a) *7-Acétoxy-8-hydroxy-p-menthane*

Un mélange de 258 g (1,5M) de 7,8-dihydroxy-p-menthane, 2700 ml d'anhydride acétique et 810 ml de pyridine a été maintenu sous agitation pendant 45 min, puis le tout a été versé sur de la glace (1 kg) et de l'eau (1000 ml). Après extraction à l'éther (3×700 ml), les phases organiques réunies ont été lavées avec du NaOH à 10% (3×500 ml), de l'eau (2×500 ml) et une solution saturée de NaCl (500 ml).

Le solvant a été évaporé et le résidu (284 g) a été distillé pour fournir une huile incolore (276 g) ayant: Eb. 108-110°/0,133 Pa (rend. 86%).

SM: $M^+ = 214$; m/e: 43, 59, 81, 96.

b) *7-Acétoxy-8-p-menthène*

Une solution de 60 g (0,28M) de l'hydroxy-acétate obtenu sous letre a ci-dessus dans 60 ml d'hexane a été versée au sommet d'une colonne en pyrex (longueur 30 cm; diamètre int. 2 cm) remplie aux deux tiers d'un mélange de γ-alumine (2,5 g) et de quartz (50 g), colonne chauffée à 250° et traversée par un débit d'azote de 100 ml/min. Les produits formés sont condensés à la sortie dans deux trappes en série refroidies à −70°. 140 ml d'hexane ont été versés au contenu des deux trappes, puis, une fois séparées, les phases organiques ont été séchées sur du $MgSO_4$. Par évaporation du solvant, on a obtenu 51,6 g d'un mélange 92:8 de 7-acétoxy-8-p-menthène et de 7-acétoxy-4(8)-p-menthène (rend. 82%).

SM: $M^+ = 196$; m/e: 43, 93, 107, 79.

c) *7-Acétoxy-8,9-époxy-p-menthane*

Un mélange d'acide peracétique 40% (34 g) et d'acétate de sodium (7 g) a été ajouté goutte à goutte (env. 2 h) et sous agitation à un mélange de 25 g du produit obtenu sous lettre b ci-dessus, 40 g de carbonate de sodium et 300 ml de chlorure de méthylène. Le mélange de réaction a été ensuite maintenu sous agitation pendant 5 h, puis il a été filtré. Le filtrat clair a été lavé successivement à l'eau, avec une solution de $Na_2SO_3$ à 10% et enfin avec de l'eau. Après concentration sous pression réduite, on a obtenu 23,2 g du produit brut désiré (pureté 88%) sous la forme d'un mélange isomérique cis/trans 75/25 (rend. 80%). Ce mélange peut être utilisé directement pour l'étape suivante de réaction.

SM: $M^+ = 212$; m/e: 43, 95, 41, 79.

d) *1(7)-p-Menthène-9-al*

Une solution de 5 g du produit obtenu sous lettre c ci-dessus dans 100 ml d'hexane a été introduite au moyen d'une pompe à piston à la vitesse de 0,5 ml/min dans une colonne de quartz vide (longueur 400 cm; diamètre int. 0,8 cm) chauffée à 500° et maintenue sous un débit d'azote de 13 ml/min. Les vapeurs obtenues à la sortie de la colonne sont ensuite condensées dans deux trappes en série refroidies à −70°, la première d'entre elles contenant 1 g de bicarbonate de sodium. Le pyrolysat a été ensuite lavé successivement avec une solution aqueuse à 5% de bicarbonate de sodium, de l'HCl à 5%, de l'eau et une solution saturée de NaCl. Après séchage sur du $Na_2SO_4$ la phase organique a été concentrée.

Quatre autres charges de 7-acétoxy-8,9-époxy-p-menthane ont été pyrolysées de la sorte. On a ainsi obtenu 15,5 g d'un mélange brut contenant 40% du menthénal désiré. Par distillation à l'aide d'une colonne Vigreux, on a pu enrichir à 60% le contenu en menthénal du mélange. Ce contenu peut d'ailleurs être ultérieurement augmenté jusqu'à 95%, ou plus, par une distillation fractionnée à l'aide d'une colonne du type de Fischer. Un échantillon analytique a pu être préparé par distillation fractionnée. Les caractères analytiques du produit obtenu étaient les suivants:

RMN ($CDCl_3$): 9,1; 8,96; 7,95-8,35; 7,76; 5,41 δ ppm;

SM: $M^+ = 152$; m/e 79, 84, 94, 41;

IR: 2940, 1720, 1655, 1455, 900 $cm^{-1}$.

Le 7,8-dihydroxy-p-menthane, utilisé comme produit de départ dans le procédé décrit ci-dessus, a été préparé conformément au procédé illustré plus haut à partir de β-pinène. La première étape, qui consiste en l'époxydation de ce dernier, a été effectuée suivant J.K. Crandall et Luan-Ho C. Lin, «Org. Chem.», *33*, 2375 (1968). L'époxyde ainsi obtenu a été ensuite traité avec de l'eau saturée au $CO_2$ sous vigoureuse agitation. Par extraction à l'éther et distillation des parties volatiles, on a obtenu le 7,8-dihydroxy-p-menthène avec un rendement d'environ 90%, Eb. 70-115°/1,33 Pa. Par hydrogénation catalytique dudit composé, on a enfin pu obtenir le 7,8-dihydroxy-p-menthane désiré.

*Exemple 2:*

Une composition parfumante de base de type

jacinthe a été préparée en mélangeant les ingrédients suivants (parties en poids):

| | |
|---|---|
| Alcool cinnamique | 2150 |
| Phénéthylol | 2265 |
| Acétate de benzyle | 1200 |
| Jasmonate de méthyle | 650 |
| Méthylisoeugénol | 650 |
| Hexylcinnamaldéhyde | 1250 |
| Terpinéol | 250 |
| Hydroxycitronellal synth. | 350 |
| Essence de galbanum à 10%* | 325 |
| Indol à 10%* | 300 |
| Pipol à 10%* | 200 |
| Acétate de pipol à 10%* | 100 |
| Aldéhyde phénylacétique à 10%* | 250 |
| Total | 9940 |

* dans le diéthylphtalate

En ajoutant à la composition ci-dessus 60 g de 1(7)-p-menthène-9-al, on obtient une nouvelle composition possédant une fraîcheur et un montant naturels. L'addition dudit menthénal sert en outre à développer la note de tête verte et le caractère fleuri de la composition.

*Exemple 3:*

Une composition parfumante de base de type fougère a été préparée en mélangeant les ingrédients suivants (parties en poids):

| | |
|---|---|
| Salicylate d'amyle | 1600 |
| Essence de bergamote synth. | 3700 |
| Coumarine | 500 |
| Essence de géranium | 400 |
| Essence de lavande | 1000 |
| Mousse de chêne à 10%* | 2750 |
| Total | 9950 |

* dans le dipropylèneglycol

En ajoutant à la composition ci-dessus 50 g de 1(7)-p-menthène-9-al, on obtient une nouvelle composition possédant un caractère chaud, aromatique et épicé qui rappelle le cumin ou le carvi.

## Revendications

1. 1(7)-p-Menthène-9-al de formule:

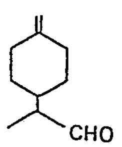

(I)

2. Procédé pour la préparation de 1(7)-p-menthène-9-al selon la revendication 1, caractérisé en ce qu'on

a) traite le 7,8-dihydroxy-p-menthane avec un agent d'acétylation pour fournir le 7-acétoxy-8-hydroxy-p-menthane,

b) déshydrate ce dernier afin d'obtenir le 7-acétoxy-8-p-menthène,

c) époxyde le composé ainsi obtenu pour donner le 7-acétoxy-8,9-époxy-p-menthane, et

d) soumet enfin cet époxyde à un traitement thermique pour fournir un pyrolysat constitué pour l'essentiel du 1(7)-p-menthène-9-al désiré.

3. Utilisation de 1(7)-p-menthène-9-al selon la revendication 1, en tant qu'ingrédient parfumant et aromatisant.

4. Composition parfumante résultant de l'utilisation selon la revendication 3, caractérisée en ce qu'elle contient de 0,01 à 0,5% en poids de 1(7)-p-menthène-9-al, des coïngrédients parfumants, des supports et/ou des solvants.

## Patentansprüche

1. 1(7)-p-Menthen-9-al der Formel

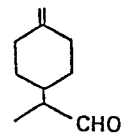

(I)

2. Verfahren zur Herstellung von 1(7)-p-Menthen-9-al gemäss Anspruch 1, dadurch gekennzeichnet, dass man

a) 7,8-Dihydroxy-p-menthan mit einem Acetylierungsmittel behandelt, um 7-Acetoxy-8-hydroxy-p-menthan zu erhalten,

b) dieses Produkt dehydratisiert, um 7-Acetoxy-8-p-menthen zu erhalten,

c) die so erhaltene Verbindung epoxydiert, um 7-Acetoxy-8,9-epoxy-p-menthan zu erhalten, und

d) dieses Epoxyd thermisch behandelt, um ein Pyrolysat zu erhalten, das hauptsächlich das gewünschte 1(7)-p-Menthen-9-al enthält.

3. Verwendung von 1(7)-p-Menthen-9-al gemäss Anspruch 1 als Parfüm- und Aromabestandteil.

4. Parfümkomposition folgend der Verwendung gemäss Anspruch 3, dadurch gekennzeichnet, dass sie 0,01 bis 0,5 Gew.-% 1(7)-p-Menthen-9-al, Parfümzusatzbestandteile, Träger und/oder Lösungsmittel enthält.

## Claims

1. 1(7)-p-Menthen-9-al of formula

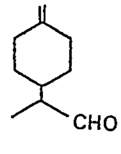

(I)

2. Process for the preparation of 1(7)-p-menthen-9-al according to Claim 1, characterized in that

(*a*) 7,8-dihydroxy-p-menthane is treated with an acetylation agent to give 7-acetoxy-8-hydroxy-p-menthane,

(*b*) this latter is dehydrated to give 7-acetoxy-8-p-menthene,

(*c*) the thus obtained compound is epoxidised to give 7-acetoxy-8,9-epoxy-p-menthane, and

(*d*) this epoxide is thermally treated to yield a

pyrolysate essentially consisting of desired 1(7)-p-menthen-9-al.

3. Utilisation of 1(7)-p-menthen-9-al according to Claim 1 as perfuming and flavouring ingredient.

4. Perfume composition resulting from the utilisation according to Claim 3, characterized in that it contains from 0.01 to 0.5% by weight of 1(7)-p-menthen-9-al, in conjunction with perfume coingredients, supports and/or solvents.